# EUROPEAN PATENT SPECIFICATION

(11) **EP 2 862 577 B1**
(45) Date of publication and mention of the grant of the patent: **02.08.2017**
(21) Application number: 13761384.0
(22) Date of filing: 08.03.2013
(51) Int. Cl.: A61K 38/22, C07K 14/63, A61P 21/00

(54) **USE OF OBESTATIN FOR MUSCLE REGENERATION**
VERWENDUNG VON OBESTATIN ZUR MUSKELREGENERATION
UTILISATION DE L'OBESTATINE POUR LA RÉGÉNÉRATION MUSCULAIRE

(30) Priority: 12.03.2012 ES 201230367
(43) Date of publication of application: 22.04.2015
(73) Proprietor: Universidade de Santiago de Compostela, 15782 Santiago de Compostela (a Coruña) (ES); Servizo Galego De Saúde (Sergas), 15703 Santiago de Compostela , A Coruña (ES)
(72) Inventor: PÉREZ CAMIÑA, Jesús, E-15782 Santiago de Compostela (ES); CASANUEVA FREIJO, Felipe, E-15782 Santiago de Compostela (ES)
(74) Representative: Pons
(86) International application number: PCT/ES2013/070149
(87) International publication number: WO 2013/135928

(56) References cited:
- WO-A1-2006/096847
- WO-A2-2009/033794
- WO-A2-2009/033795
- ALESSANDRA BARAGLI ET AL: "Neuroendocrine and metabolic activities of ghrelin gene products", PEPTIDES, ELSEVIER, AMSTERDAM, NL, vol. 32, no. 11, 25 October 2011 (2011-10-25), pages 2323-2332, XP028114484, ISSN: 0196-9781, DOI: 10.1016/J.PEPTIDES.2011.10.024 [retrieved on 2011-10-30]
- G. ALLOATTI ET AL: "Obestatin affords cardioprotection to the ischemic-reperfused isolated rat heart and inhibits apoptosis in cultures of similarly stressed cardiomyocytes", AJP: HEART AND CIRCULATORY PHYSIOLOGY, vol. 299, no. 2, 1 August 2010 (2010-08-01), pages H470-H481, XP055216721, ISSN: 0363-6135, DOI: 10.1152/ajpheart.00800.2009
- GRANATA RICCARDA ET AL: "Cardiovascular actions of the ghrelin gene-derived peptides and growth hormone-releasing hormone", EXPERIMENTAL BIOLOGY AND MEDICINE (MAYWOOD), vol. 236, no. 5, May 2011 (2011-05), pages 505-514, XP009186297, ISSN: 1535-3702
- D. MONTOYA-FLORES ET AL: "Ghrelin stimulates myogenic differentiation in a mouse muscle satellite cell line and in primary cultures of bovine myoblasts", JOURNAL OF ANIMAL PHYSIOLOGY AND ANIMAL NUTRITION, vol. 96, no. 4, 21 July 2011 (2011-07-21), pages 725-738, XP055217856, ISSN: 0931-2439, DOI: 10.1111/j.1439-0396.2011.01201.x
- BANG, A.S. ET AL.: 'Characterisation of proghrelin peptides in mammalian tissue and plasma' JOURNAL OF ENDOCRINOLOGY. vol. 192, no. 2, 01 February 2007, pages 313 - 323, XP055165755
- ZHANG, J.V. ET AL.: 'Obestatin, a peptide encoded by the ghrelin gene, opposes ghrelin's effects on food intake' SCIENCE. vol. 310, no. 5750, 11 November 2005, pages 996 - 999, XP002373204
- SOARES, J.B. ET AL. GHRELIN, DES-ACYL GHRELIN AND OBESTATIN: THREE PIECES OF THE SAME PUZZLE vol. 29, no. 7, 01 July 2008, pages 1255 - 1270, XP022704828
- GURRIARAN-RODRIGUEZ, U. ET AL.: 'The obestatin/GPR39 system is up-regulated by muscle injury and functions as a autocrine regenerative system' THE JOURNAL OF BIOLOGICAL CHEMISTRY vol. 287, no. 45, 02 November 2012, pages 38379 - 38389, XP055165760

### SEQUENCE LISTING

<110> Universidade de Santiago de Compostela Servizo Galego de Saude (SERGAS)
<120> Use of obestatin for muscle regeneration
<130> ES1596.40
<140> P201230367
   <141> 2012-03-12
<160> 12
<170> PatentIn version 3.5
<210> 1
   <211> 23
   <212> PRT
   <213> Mus musculus
<400> 1
   Phe Asn Ala Pro Phe Asp Val Gly Ile Lys Leu Ser Gly Ala Gln Tyr
   1               5                   10                  15
   Gln Gln His Gly Arg Ala Leu
               20
<210> 2
   <211> 69
   <212> DNA
   <213> Mus musculus
<400> 2
   ttcaatgctc ccttcgatgt tggcatcaag ctgtcaggag ctcagtatca gcagcatggc      60
   cgggccctg                                                              69
<210> 3
   <211> 23
   <212> PRT
   <213> Homo sapiens
<400> 3
   Phe Asn Ala Pro Phe Asp Val Gly Ile Lys Leu Ser Gly Val Gln Tyr
   1               5                   10                  15
   Gln Gln His Ser Gln Ala Leu
               20
<210> 4
   <211> 69
   <212> DNA
   <213> Homo sapiens
<400> 4
   ttcaacgccc cctttgatgt tggaatcaag ctgtcagggg ttcagtacca gcagcacagc    60
   caggccctg                                                            69
<210> 5
   <211> 117
   <212> PRT
   <213> Homo sapiens
<400> 5
   Met Pro Ser Pro Gly Thr Val Cys Ser Leu Leu Leu Leu Gly Met Leu
   1 5 10 15
   Trp Leu Asp Leu Ala Met Ala Gly Ser Ser Phe Leu Ser Pro Glu His
   20 25 30
   Gln Arg Val Gln Gln Arg Lys Glu Ser Lys Lys Pro Pro Ala Lys Leu
   35 40 45
   Gln Pro Arg Ala Leu Ala Gly Trp Leu Arg Pro Glu Asp Gly Gly Gln
   50 55 60
   Ala Glu Gly Ala Glu Asp Glu Leu Glu Val Arg Phe Asn Ala Pro Phe
   65 70 75 80
   Asp Val Gly Ile Lys Leu Ser Gly Val Gln Tyr Gln Gln His Ser Gln
   85 90 95
   Ala Leu Gly Lys Phe Leu Gln Asp Ile Leu Trp Glu Glu Ala Lys Glu
   100 105 110
   Ala Pro Ala Asp Lys
   115
<210> 6
   <211> 354
   <212> DNA
   <213> Homo sapiens
<400> 6
   atgccctccc cagggaccgt ctgcagcctc ctgctcctcg gcatgctctg gctggacttg 60
   gccatggcag gctccagctt cctgagccct gaacaccaga gagtccagca gagaaaggag 120
   tcgaagaagc caccagccaa gctgcagccc cgagctctag caggctggct ccgcccggaa 180
   gatggaggtc aagcagaagg ggcagaggat gaactggaag tccggttcaa cgcccccttt 240
   gatgttggaa tcaagctgtc aggggttcag taccagcagc acagccaggc cctggggaag 300
   tttcttcagg acatcctctg ggaagaggcc aaagaggccc cagccgacaa gtga 354
<210> 7
   <211> 117
   <212> PRT
   <213> Mus musculus
<400> 7
   Met Leu Ser Ser Gly Thr Ile Cys Ser Leu Leu Leu Leu Ser Met Leu
   1               5                   10                  15
   Trp Met Asp Met Ala Met Ala Gly Ser Ser Phe Leu Ser Pro Glu His
               20                  25                  30
   Gln Lys Ala Gln Gln Arg Lys Glu Ser Lys Lys Pro Pro Ala Lys Leu
           35                  40                  45
   Gln Pro Arg Ala Leu Glu Gly Trp Leu His Pro Glu Asp Arg Gly Gln
       50                  55                  60
   Ala Glu Glu Thr Glu Glu Glu Leu Glu Ile Arg Phe Asn Ala Pro Phe
   65                  70                  75                  80
   Asp Val Gly Ile Lys Leu Ser Gly Ala Gln Tyr Gln Gln His Gly Arg
                   85                  90                  95
   Ala Leu Gly Lys Phe Leu Gln Asp Ile Leu Trp Glu Glu Val Lys Glu
               100                 105                 110
   Ala Pro Ala Asp Lys
           115
<210> 8
   <211> 354
   <212> DNA
   <213> Mus musculus
<400> 8
   atgctgtctt caggcaccat ctgcagtttg ctgctactca gcatgctctg gatggacatg      60
   gccatggcag gctccagctt cctgagccca gagcaccaga aagcccagca gagaaaggaa     120
   tccaagaagc caccagctaa actgcagcca cgagctctgg aaggctggct ccacccagag     180
   gacagaggac aagcagaaga gacagaggag gagctggaga tcaggttcaa tgctcccttc     240
   gatgttggca tcaagctgtc aggagctcag tatcagcagc atggccgggc cctggggaag     300
   tttcttcagg atatcctctg ggaagaggtc aaagaggcgc cagctgacaa gtaa           354
<210> 9
   <211> 23
   <212> PRT
   <213> Bubalus bubalis
<400> 9
   Phe Asn Ala Pro Phe Asn Ile Gly Ile Lys Leu Ser Gly Ala Gln Ser
   1               5                   10                  15
   Leu Gln His Gly Gln Leu Thr
               20
<210> 10
   <211> 23
   <212> PRT
   <213> Cricetulus griseus
<400> 10
   Phe Asn Ala Pro Phe Asp Val Gly Ile Lys Leu Ser Gly Ala Gln Tyr
   1               5                   10                  15
   Gln Gln His Gly Gln Ala Leu
               20
<210> 11
   <211> 23
   <212> PRT
   <213> Ailuropoda melanoleuca
<400> 11
   Phe Asn Ala Pro Phe Asp Val Gly Ile Lys Leu Ser Gly Ala Gln Tyr
   1               5                   10                  15
   Gln Glu His Gly Gln Ala Leu
               20
<210> 12
   <211> 23
   <212> PRT
   <213> Felis catus
<400> 12
   Phe Asn Ala Pro Phe Asp Val Gly Ile Lys Leu Ser Gly Ala Gln Tyr
   1               5                   10                  15
   His Gln His Gly Gln Ala Leu
               20

## Description

The present invention is included in the field of medicine, specifically within that of peptides with useful myogenic capacity to induce muscle regeneration and, therefore, for the treatment and/or prevention of injuries or diseases which present muscle damage.

### PRIOR ART

Myogenesis is the process of muscle tissue formation that involves the specification and differentiation of precursor cells of muscle or myoblasts, their fusion for the formation of primary and secondary myotubes and the consequent maturation in myofibers (Charge SB., et al., 2004, Physiol Rev. 84:209-238). This process is required both for the development of the skeletal muscle in the embryo and for the postnatal growth and maintenance and repair of the myofibers of the skeletal muscle after an injury (Perry RL., et al., 2000, Front Biosci. 5:D750-767; Sartorelli V., et al., 2005, Curr Opin Genet Dev. 15(5):528-533).

Therefore, the muscle fibres or myocytes are formed from the fusion of myoblasts in multinucleated fibres, called myotubes. In the embryonic development the myogenesis process starts with the proliferation of myoblasts, which proliferate if there is sufficient fibroblast growth factor (FGF). On the contrary, in the absence of FGF, the myoblasts stop their division and secrete fibronectin to the extracellular matrix. The second stage involves the alignment of the myoblasts to form myotubes. The third stage consisting of a cell fusion, wherein the calcium ions are critical, which is mediated by a set of metalloproteinases called meltrins.

The Myocyte Enhance Factors (MEFs) promote myogenesis. Furthermore, the Serum Response Factor (SRF) plays a vital role during myogenesis, being necessary for the expression of actin alpha genes in the striated muscle. The expression of skeletal actin alpha is also regulated by the androgen receptor. Thus, the spheroids can regulate myogenesis.

In the cicatrization process, the myoblasts are activated early, regenerating muscle by the generation of new fibres or fusion with pre-existing ones. The myoblasts induced to proliferate in response to various growth factors express the myogenin transcription factor which promotes the expression of specific proteins of the skeletal muscle, such as creatine kinase and myosin.

There are different molecular markers which allow the detailed analysis of myogenic determination, myoblast proliferation and terminal differentiation. The myogenic regulatory factors are vital for the determination and maintenance of the skeletal muscle. In this sense, during development, the induction of MyoD and Myf-5 expression defines the origin of the myogenic progenitor cells which are responsible for forming different muscle groups in the adult organism. There is a high number of signalling pathways involved in regulating myogenesis during the development and regeneration of the damaged muscle tissue. These pathways regulate the progression of the cell cycle, the protein-protein interactions and the transcriptional activity of the myogenic factors.

The myogenesis process requires the coordinated actions of multiple signalling pathways which regulate the output of the cell cycle, determining and specifying the myogenesis process based on myogenic expression factors, a group of transcription factors including Myf-5, MyoD, myogenin and MRF4.

Furthermore, a gastrointestinal peptide with 23 amino acids has been identified, derived from the same precursor peptide of ghrelin, pre-proghrelin, called obestatin. Obestatin was originally isolated from the stomach, demonstrating that it was a circulating peptide with pulsatile secretion, showing an ultradian rhythm similar to that of ghrelin and growth hormone (GH) (Zhang JV., et al., 2005, Science 310:996-999). Mitogenic actions (Camiña JP., et al., 2007, J Cell Physiol. 21 1: 1-9) and adipogenic actions (Gurriarán-Rodríguez U., et al., 2010, J Cell Mol Med. 15(9): 1927-40) have been described for obestatin, which shows a biological functionality for this peptide. Recently, it has been described that obestatin promotes cell survival in β cells and in human pancreatic islet cells. Likewise, obestatin induces the expression of genes which regulate the destination of the β cells, insulin biosynthesis and glucose sensitivity (Granata R., et al., 2008, Diabetes 57:967-979).

In summary, damaged muscle tissue requires a myogenic process for its maintenance and regeneration, so that the agents capable of promoting said myogenesis, and therefore stimulating the regeneration of the muscle myofibers, are of special clinical interest due to their potential application in the treatment and/or prevention of diseases and injuries which present muscle damage.

### DESCRIPTION OF THE INVENTION

The present invention is defined in the claims. The present invention provides an isolated peptide called "obestatin" which promotes the proliferation and differentiation in myoblasts. The proliferative effect in myoblasts shows a clear dose-dependent pattern. The role as myogenic factor is established through the expression in myoblasts of key factors for their differentiation, such as, for example, but without limitation, the early differentiation marker, myogenin, and the late differentiation marker, myosin, (MHC- "Myosin Skeletal Heavy Chain"), in dose-dependent manner. Thus, this peptide exercises a role as myoblast survival factor extending the regenerative potential of the muscle system through the increase in proliferative activity of the myoblasts. On the other hand, it promotes myogenesis through the exit from the cell cycle facilitating the migration, the differentiation and the fusion of the myoblasts for the determination of myotubes. Therefore, said peptide is useful in muscle regeneration, preferably in skeletal muscle regeneration, thus being useful in the treatment and/or prevention of diseases or injuries which present muscle damage. There are obestatins in different organisms such as, for example, but without limitation, mouse, rat, buffalo, bear, cat or human which share a high sequence identity and having the same capacity of promoting myogenesis.

Therefore, a first aspect of the invention relates to the use of an isolated peptide comprising an amino acid sequence comprising the SEQ ID NO: 3, or of the nucleotide sequence encoding it, as in vitro myogenic agent in skeletal muscle. "Obestatin" in the present disclosure is understood as an amino acid sequence with at least 86% identity with SEQ ID NO: 1. Human obestatin is understood as a peptide of sequence SEQ ID NO:3 which has a homology greater than 86% with SEQ ID NO: 1. Mouse obestatin or rat obestatin is understood as the peptide of sequence SEQ I D NO: 1.

The amino acid sequence of mouse obestatin, and which coincides with rat obestatin, is SEQ ID NO: 1. This sequence SEQ ID NO:1 corresponds to residues 76 to 98 of the sequence with NCBI access number (National Center for Biotechnology Information) NP_067463 corresponding to a pre-propeptide called pre-proghrelin of sequence SEQ ID NO:7, which, when it is processed in the organism, gives rise to obestatin of sequence SEQ ID NO: 1. This propeptide is encoded by the ghrelin gene.

In the examples of the present disclosure, rat/mouse obestatin was used since the corresponding tests were carried out on rat myoblast cell lines as well as on the rats themselves, and therefore it was necessary to have the correspondence between the test sequence and the respective target in the tests. However, it must be understood that the most useful application of the present invention and its ultimate aim is its therapeutic use in humans. In this sense, it can be expected that human obestatin, a homologous element to that of rat/mouse obestatin with a high percentage of identity in its amino acid sequence with respect to rat/mouse amino acid sequence, and with just 3 amino acids difference (86.9% identity), also exercises a comparable effect as myogenic agent, both on the corresponding lines of human myoblasts and forming part of a medicament for muscle regeneration in a human being. In the present invention, human obestatin corresponds to the sequence SEQ ID NO:3. This sequence is relates to the residues 76 to 98 of the sequence with NCBI access number NP_057446 corresponding to the pre-proghrelin of sequence SEQ ID NO:5, which is processed in the organism giving rise to obestatin of sequence SEQ ID NO:3.

The peptide of the invention may be encoded by a nucleotide sequence which takes place directly by its transcription-translation to the peptide of 23 amino acids. An example of sequence directly encoding mouse or rat obestatin (SEQ ID NO: 1) could be, but without limitation, the sequence SEQ ID NO: 2, so that preferably, the nucleotide sequence referred to in the present disclosure and encoding the mouse obestatin is SEQ ID NO: 2.

On the other hand, an example of sequence directly encoding human obestatin (SEQ ID NO:3), could be, for example, but without limitation, the sequence SEQ ID NO:4. Therefore, preferably, the nucleotide sequence encoding human obestatin is SEQ ID NO: 4.

Obestatin in organisms is encoded by the ghrelin gene. This gene gives rise to a peptide called pre-proghrelin. In humans this peptide has as sequence SEQ ID NO: 5, and is processed in the organism giving obestatin as product. Specifically, in humans, pre-proghrelin has 117 amino acids and its sequence would be SEQ ID NO:5. The encoding sequence without introns for human pre-proghrelin, and, therefore, encoding a peptide comprising human obestatin would be the sequence SEQ ID NO:6, which corresponds to the nucleotide sequence of 354 base pairs with access number CCDS33700.1, in the Consensus CDS (CCDS) database. Therefore, preferably, the nucleotide sequence encoding human obestatin is SEQ ID NO: 6. Furthermore, in mouse, the encoding sequence without introns for pre-proghrelin of 117 amino acids (SEQ ID NO:7), and which therefore also encode a peptide comprising obestatin, would be the sequence SEQ ID NO:8, which corresponds to the nucleotide sequence of 354 base pairs with access number CCDS20432.1 in the Consensus CDS (CCDS) database. Therefore, preferably, the nucleotide sequence encoding the mouse obestatin is SEQ ID NO: 8.

The term "% identity" with respect to a polypeptide or protein relates to the percentage of amino acids of the sequence in question which are identical to the amino acids of the sequence it is compared with, after aligning said sequences and introducing spaces, if necessary, to achieve the maximum percentage of identity without having to consider the conservative substitutions. The alignment could be performed in different ways by the person skilled in the art, such as, for example, using public tools such as the BLAST, BLAST-2, ALIGN or Megalign (DNASTAR) programs. A person skilled in the art can determine the appropriate parameters to measure the alignment, including the necessary algorithms to achieve the maximum alignment of the sequences compared. In the present disclosure, the% of identity is calculated by dividing the number of amino acids which are identical after aligning SEQ ID NO: 1 and the candidate sequence, by the total number of amino acids of SEQ ID NO: 1, and multiplying the result by 100. In the case of a nucleotide sequence, the "% of identity" is calculated in the same way although nucleotides are compared.

**Table 1. Percentages of identity between the amino acid sequences of mouse and rat obestatin in comparison with the human sequence and for other animals.**

| | Protein | | |
|---|---|---|---|
| Species | % identity | Identical amino acids | Sequence |
| *Mus musculus* | 100 | 23/23 | LARGHQQYQAGSLKIGVDFPANF (SEQ ID NO:1) |
| *Rattus Norvegicus* | 100 | 23/23 | LARGHQQYQAGSLKIGVDFPANF (SEQ ID NO:1) |
| *Homo sapiens* | 86.9 | 20/23 | LAQSHQQYQVGSLKIGVDFPANF (SEQ ID NO:3) |
| *Bubalus bubalis* | 74 | 17/23 | LTQGHQLSQAGSLKIGINFPANF (SEQ ID NO:9) |
| *Cricetulus griseus* | 95.6 | 22/23 | LAQGHQQYQAGSLKIGVDFPANF (SEQ ID NO:10) |
| *Ailuropoda melanoleuca* | 91.3 | 21/23 | LAQGHEQYQAGSLKIGVDFPANF (SEQ ID NO:11) |
| *Felis catus* | 91.3 | 21/23 | LAQGHQHYQAGSLKIGVDFPANF (SEQ ID NO:12) |

Therefore, herein disclosed is the use of an isolated peptide comprising an amino acid sequence with at least 86% identity with SEQ ID NO: 1, wherein the amino acid sequence with at least 86% identity with SEQ ID NO: 1 is SEQ ID NO:3, or to the use of the nucleotide sequence encoding it, as *in vitro* myogenic agent. Since SEQ ID NO:3 corresponds to human obestatin, and in the organism it is synthesized in the form of pre-proghrelin, which is processed giving rise to obestatin, this pre-proghrelin would have the same utility as obestatin. Therefore, preferably, the amino acid sequence comprising SEQ ID NO:3 is human pre-proghrelin (SEQ ID NO:5). Furthermore, herein disclosed is the use of an isolated peptide which consisting of an amino acid sequence with at least 86% identity with SEQ ID NO: 1, where the amino acid sequence with at least 86% identity with SEQ ID NO: 1 is SEQ ID NO:3, or to the use of the nucleotide sequence encoding it, as *in vitro* myogenic agent. Herein disclosed is the use of an isolated peptide comprising an amino acid sequence with at least 91% identity with SEQ ID NO: 1, or to the use of the nucleotide sequence encoding it, as *in vitro* myogenic agent. Further disclosed is the use of an isolated peptide comprising an amino acid sequence with at least 95% identity with SEQ ID NO: 1, or to the use of the nucleotide sequence encoding it, as *in vitro* myogenic agent. An even more preferred embodiment relates to the use of an isolated peptide comprising the amino acid sequence SEQ ID NO: 1, or to the use of the nucleotide sequence encoding it, as *in vitro* myogenic agent. Since SEQ ID NO:1 corresponds to mouse or rat obestatin, and in the organism it is synthesized in the form of pre-proghrelin, which is processed giving rise to obestatin, this pre-proghrelin would have the same utility as obestatin. Therefore, preferably the amino acid sequence comprising SEQ ID NO:1 is mouse or rat pre-proghrelin (SEQ ID NO:7). Also herein disclosed is the use of an isolated peptide which consisting of the amino acid sequence SEQ ID NO: 1, or to the use of the nucleotide sequence encoding it, as *in vitro* myogenic agent.

Obestatin and variants or derivatives thereof may be synthesized, for example, although without limitation, by chemical synthesis, recombinant DNA techniques, isolation of natural sources or by *in vitro* proteolysis. Obestatin may be produced recombinantly, not only directly but as a fusion polypeptide together with a heterologous polypeptide, which may contain, for example, although without limiting ourselves, a signal sequence, or another polypeptide which has a cut-off site for a protease, for example, although without limiting ourselves, at the N-terminal end of the mature protein or of the polypeptide.

The terms "nucleotide sequence", "sequence of nucleotides", "nucleic acid", "oligonucleotide" and "polynucleotide" are used here interchangeably and relate to a polymeric form of nucleotides of any length which may or may not be chemically or biochemically modified. They, therefore, relate to any polyribonucleotide or polydeoxyribonucleotide, single chain and two-strand. Any of the sequences of the present invention, and which can encode, for example, but without limitation to, obestatin, may be obtained artificially using methods of cloning and conventional selection, or by sequencing. Said nucleotide sequence, additionally to the coding sequence, may have other elements, such as, for example, although without limiting ourselves, introns, non-coding sequences at ends 5' and/or 3', binding sites to ribosomes, or stabilizing sequences. These polynucleotides may additionally including encoding sequences for additional amino acids which may be useful, for example, but without limitation, for increasing the stability of the peptide generated from them or to allow a better purification thereof.

In another preferred embodiment, the nucleotide sequence encoding obestatin is included in an expression vector.

An "expression vector" is a molecule of nucleic acid used to transfer genetic material to a cell. Apart from said genetic material, a vector may also contain different functional elements which include transcription control elements, such as, for example, although without limiting ourselves, promoters or operators, regions or enhancers of the binding to transcription factors and control elements to start and end the translation. The vectors include, but without limitation: plasmids, cosmids, virus, phages, recombinant expression cassettes and transposones. Some vectors are capable of autonomously replicating or dividing once they are introduced in the host cell, such as bacterial vectors with a bacterial replication source or mammalian episomal vectors. An expression vector is that capable of directing the expression of genes that have been operatively bound. An expression vector is used for the transcription and the translation of a gene of interest, normally controlled by a promoter. A "promoter" is a sequence of nucleotides which controls the translation of the gene of interest. The promoter is operatively bound to the gene of interest. "Operatively bound" relates to the functional relationship and the location of the promoter's sequence with respect to the gene of interest.

Obestatin, or the nucleotide sequence encoding it, can be used as *in vitro* myogenic agents, being expressed in a cell culture, preferably of myoblasts, to induce the differentiation of the cells under culture, so that they form myotubes (multinucleated syncytial cells, with nuclei in central position, but without myotic activity) and, later, muscle fibres or myocytes which can later be used in somatic cell therapy procedures for the regeneration of damaged muscle tissue, preferably skeletal muscle tissue. Therefore, obestatin, the nucleotide sequence encoding it and the cultivated cells, preferably myoblasts, which are intended to be differentiated by this *in vitro* myogenesis process are, more preferably of human origin and even more preferably of autologous origin.

As used here, the term "myogenic agent" relates to that agent capable of inducing the proliferation, and/or differentiation of a pluri- or multipotential cell, preferably of a myoblast, to a myocyte or muscle fibre, i.e. a fusiform and multinuclear with contractile capacity and which forms part of the muscle tissue.

A "myoblast" is an undifferentiated cell with capacity to synthesize fine filaments just before their fusion process with other myoblasts and give rise to the formation of myotubes. Myoblasts are cells with high capacity for cell division and constitute the germinated cells of the muscle.

Likewise, obestatin or the nucleotide sequence encoding it may be administered to an individual who suffers an injury or disease which presents damage in the muscle tissue, preferably in the skeletal muscle tissue, to promote muscle regeneration and thus re-establish the pathological condition. Another aspect of the invention relates to an isolated peptide comprising an amino acid sequence comprising the SEQ ID NO: 3, or the nucleotide sequence encoding it for use in the treatment and/ or prevention of skeletal muscle injuries or skeletal muscle diseases selected from the list comprising: neurogenic myopathies, muscular necrosis, muscular atrophy, sprains, distensions, cramps, tendonitis, contractures, muscular dystrophy, myositis, muscle infections, myofascial pain, metabolic myopathies, muscle twitching, hypotonia, congenital myopathies, muscle paralysis, rhabdomyolysis or compartment syndrome. Further disclosed is an isolated peptide consisting of an amino acid sequence with at least 86% identity with SEQ ID NO: 1. In a more preferred embodiment, the amino acid sequence with at least 86% identity with SEQ ID NO: 1 is SEQ ID NO:3. Also disclosed is an isolated peptide which comprises an amino acid sequence with at least 91% identity with SEQ ID NO: 1. Further disclosed is an isolated peptide comprising an amino acid sequence with at least 95% identity with SEQ ID NO: 1, as well as an isolated peptide comprising the sequence SEQ ID NO: 1. Since SEQ ID NO: 1 corresponds to mouse or rat obestatin, and in the organism it is synthesized in the form of pre-proghrelin, which is processed giving rise to obestatin, this pre-proghrelin would have the same usefulness as obestatin. Therefore, preferably, the amino acid sequence comprising SEQ ID NO: 1 is mouse or rat pre-proghrelin (SEQ ID NO:7). In an even more preferred embodiment, the isolated peptide consisting of the amino acid sequence SEQ ID NO: 1. Preferable, the nucleotide sequence encoding the isolated peptide is SEQ ID NO: 4. Alternatively, the nucleotide sequence encoding the isolated peptide is SEQ IO NO: 2 or is (SEQ ID NO: 6. Therefore, preferably, the nucleotide sequence encoding the isolated peptide is SEQ ID NO: 8. The nucleotide sequence encoding the isolated peptide may be included in an expression vector.

"Muscle regeneration" relates to the formation of muscle tissue, preferably of skeletal muscle tissue, with the aim of maintaining and/or repairing the myofibers damaged after an injury or disease which presents muscle damage, thus helping the increase, restoral or partial or total substitution of the functional activity of the damaged muscle tissue, preferably of the functional activity of the damaged skeletal muscle.

In another preferred embodiment, the medicament is for use in the treatment and/or prevention of injuries or diseases which present damage or destruction of the muscle. These diseases are, for example but without limitation, degenerative or genetic diseases which present muscle damage, preferably with damage of the skeletal muscle as well as the muscle injuries caused by a direct trauma associated to physical exercise, such as, for example, and without limitation, the trauma associated to excessive physical exercise or resistance training. There are various diseases and injuries which present muscle damage and which therefore require a regeneration of said tissue, such as, for example, although without limiting ourselves, muscular dystrophies (such as Becker muscular dystrophy or Duchenne muscular dystrophy), muscle injuries such as those produced by a direct trauma associated to physical exercise, inflammatory myopathies or myositis, distal myopathies, myotonic myopathies, congenital myopathies, mitochondrial myopathies, metabolic myopathies (such as Pompe disease, Forbes disease or Tarui disease), primary periodic paralysis, neurogenic myopathies, muscular necrosis, muscular atrophy, sprains, distensions, cramps, tendonitis, contractures (such as Volkmann's ischemic contracture or Dupuytren's contracture), muscle infections, myofascial pain, muscle twitching, hypotonia, rhabdomyolysis or compartment syndrome.

Thus, the medicament is for use in the treatment and/or prevention of muscle diseases or injuries selected from the list comprising: neurogenic myopathies, muscular necrosis, muscular atrophy, sprains, distensions, cramps, tendonitis, contractures, muscular dystrophy, myositis, muscle infections, myofascial pain, metabolic myopathies, muscle twitching, hypotonia, congenital myopathies, muscle paralysis, rhabdomyolysis or compartment syndrome. In an even more preferred embodiment, the medicament is for use in the treatment and/or prevention of muscle diseases or injuries selected from the list comprising: neurogenic myopathies, muscular necrosis, muscular atrophy or muscular dystrophy.

Muscle diseases present a limited number of symptoms, among which we can highlight weakness, tiredness, muscle pain, contractures and the alteration of walking. Furthermore, the muscle may have a variety of involuntary motor phenomenon, although most of them are a reflection of their denervation and not typically of a muscle disease, so that it is important to differentiate the clinical manifestations of myopathies from those of neuropathies. In general terms, the diagnosis is performed based on a muscle examination which involves the detection of: alterations in walking, pelvic weakness, loss of muscle mass, pseudohypertrophy, twitching and and/or myoclonus. In parallel, it assesses the consistency, tone, painfulness, strength and muscle reflex. The complementary examinations are performed based on clinical analyses (muscle enzymes and myoglubinuria), weakness tests, imaging techniques (ecography, NMR, CAT), electromyography and muscle biopsy.

Preferably, the medicament comprises obestatin, or the nucleotide sequence encoding it, in a therapeutically effective quantity, understanding as "therapeutically effective quantity" the quantity of obestatin or of nucleotide sequence encoding it which produces the desired effect. The dosage to obtain a therapeutically effective quantity depends on a variety of factors, such as, for example, the weight, sex or tolerance of the individual the medicament is going to be administered to.

Another aspect of the invention relates to the use of a pharmaceutical composition, "composition of the invention", comprising:
a. an isolated peptide comprising an amino acid sequence comprising the SEQ ID NO: 3, or
b. the nucleotide sequence encoding the peptide of (a), for use in the treatment and/
   or prevention of skeletal muscle injuries or skeletal muscle diseases selected from the list comprising: neurogenic myopathies, muscular necrosis, muscular atrophy, sprains, distensions, cramps, tendonitis, contractures, muscular dystrophy, myositis, muscle infections, myofascial pain, metabolic myopathies, muscle twitching, hypotonia, congenital myopathies, muscle paralysis, rhabdomyolysis or compartment syndrome.
An amino acid sequence with at least 86% identity with SEQ ID NO: 1 is SEQ ID NO:3. Since SEQ ID NO:3 corresponds to human obestatin, and in the organism it is synthesized in the form of pre-proghrelin, which is processed giving rise to obestatin, this pre-proghrelin would have the same usefulness as obestatin. Therefore, preferably, the amino acid sequence comprising SEQ ID NO:3 is human pre-proghrelin (SEQ ID NO:5). The isolated peptide consisting of an amino acid sequence with at least 86% identity with SEQ ID NO: 1. In a more preferred embodiment, the amino acid sequence with at least 86% identity with SEQ ID NO: 1 is SEQ ID NO:3. An isolated peptide may comprise an amino acid sequence with at least 91% identity, or at least 95% identity with SEQ ID NO: 1. An isolated peptide may further comprise the sequence SEQ ID NO: 1. Since SEQ ID NO: 1 corresponds to mouse or rat obestatin, and in the organism it is synthesized in the form of pre-proghrelin, which is processed giving rise to obestatin, this pre-proghrelin would have the same usefulness as obestatin. Therefore, preferably, the amino acid sequence comprising SEQ ID NO: 1 is mouse or rat pre-proghrelin (SEQ ID NO:7). Preferably, the isolated peptide consisting of the amino acid sequence SEQ ID NO: 1. Further disclosed is a nucleotide sequence encoding the isolated peptide which is SEQ ID NO: 4, or is SEQ ID NO: 2, or preferably is SEQ ID NO: 6. Therefore, preferably the nucleotide sequence encoding the isolated peptide is SEQ ID NO: 8. The nucleotide sequence encoding the isolated peptide may be included in an expression vector. Furthermore, herein disclosed, the medicament is for use in the treatment and/or prevention of muscle diseases or injuries selected from the list comprising: neurogenic myopathies, muscular necrosis, muscular atrophy, sprains, distensions, cramps, tendonitis, contractures, muscular dystrophy, myositis, muscle infections, myofascial pain, metabolic myopathies, muscle twitching, hypotonia, congenital myopathies, muscle paralysis, rhabdomyolysis or compartment syndrome. In another preferred embodiment, the medicament is for use in the treatment and/or prevention of muscle diseases or injuries selected from the list comprising: neurogenic myopathies, muscular necrosis, muscular atrophy or muscular dystrophy.

The composition of the disclosure may further comprise other active principle(s), excipient(s) and/or pharmaceutically acceptable vehicle(s).

The term "excipient" makes reference to a substance which helps the absorption of the elements of the composition of the disclosure, stabilizes said elements, activates or helps the manufacture of the composition in the sense of giving it consistency or providing tastes which make it more pleasant. Thus, the excipients may have the function of maintaining the ingredients together, such as, is the case of starches, sugars or celluloses, the function of sweetening, the function of colouring agent, the function of protection of the composition, such as, for example, to isolate it from the air and/or moisture, the function of filling a tablet, capsule or any other form of presentation, the disintegrating function to facilitate the dissolution of the components and their absorption in the intestine, without excluding any other type of excipients not mentioned in this paragraph.

The "pharmaceutically acceptable vehicle", the same as the excipient, is a substance which is used in the composition to dilute any of the components included therein until a determined volume or weight. The pharmaceutically acceptable vehicle is an inert substance or action analogous to obestatin and the nucleotide sequence encoding it. The function of the vehicle is to facilitate the incorporation of these elements, allow a better dosing and administration or give consistency and form to the composition.

As used here, the term "active principle", "active substance", "pharmaceutically active substance", "active ingredient" or "pharmaceutically active ingredient" means any component that potentially provides a pharmacological activity or other different effect in the diagnosis, cure, mitigation, treatment, or prevention of a disease, or which affects the structure or function of the body of humans or other animals. The term includes those components which promote a chemical change in the manufacture of the medicament and which are present therein in an anticipated modified form which provides the specific activity or the effect.

The composition of the disclosure may be formulated for its administration in a variety of forms known in the state of the art. As examples of preparations it includes any solid composition (tablets, pills, capsules, granules, etc.) or liquid (solutions, suspensions or emulsions) for oral, topical or parenteral administration. The composition of the disclosure may also be formulated in the form of liposomes, nanoparticles, nanospheres, microparticles, micelles, of sustained release formulations or any other conventional release system.

Such compositions and / or the formulations thereof may be administered to an animal, including a mammal and, therefore, a human being, in a variety of forms, including, without limitation, oral, parenteral (intraperitoneal, intravenous, intradermal, intraspinal, intrastromal, intra-articular, intrathecal, intralesional, intra-arterial, intramuscular, intranasal, subcutaneous, intracapsular), topical, using transdermal patches or by rectal route, by the administration of a suppository, percutaneous, nasal spray, surgical implant, internal surgical paint, infusion pump or via catheter.

The "medicament" referred to in the present disclosure may be for human or veterinary use. The "medicament for used in humans" is any substance or combination of substances which has properties for the treatment or prevention of diseases or injuries in human beings or which can be used in human beings or administered to human beings with the aim or restoring, correcting or modifying the physiological functions exercising a pharmacological, immunological or metabolic action, or of establishing a medical diagnosis. The "medicament for veterinary use" is any substance or combination of substances which has curative or preventative properties with respect to animal diseases or injuries or which may be administered to an animal with the aim of re-establishing, correcting or modifying its physiological functions exercising a pharmacological, immunological or metabolic action, or of establishing a veterinary diagnosis.

The medicament of the disclosure can be used both alone and in combination with other medicaments or compositions for muscle regeneration, preferably for the treatment and/or prevention of injuries or degenerative or genetic diseases which present muscle damage, more preferably of the skeletal muscle.

The term "treatment", as understood in the present disclosure, relates to combating the effects caused as a consequence of an injury or degenerative or genetic disease which present muscle damage, preferably with damage in the skeletal muscle, in a subject (preferably mammal, and more preferably human) which includes:
(i) inhibiting the disease or pathological condition, i.e. stopping its development;
(ii) alleviating the disease or the pathological condition, i.e. causing the regression of the disease or the pathological condition or its symptoms; or
(iii) stabilizing the disease or the pathological condition.

The term "prevention" as understood in the present disclosure, consisting of avoiding the appearance of the disease, i.e. avoiding that the disease or the pathological condition occurs in a subject (preferably mammal, and more preferably human), in particular, when said subject is predisposed to the pathological condition, but has not yet been diagnosed as having it.

Throughout the description and the claims the word "comprises" and its variants are not intended to exclude other technical characteristics, additives, components or steps. For persons skilled in the art, other objects, advantages and characteristics of the disclosure will be inferred in part from the description and in part from the practice of the disclosure. The following figures and examples are provided by way of illustration, and are not intended to limit the present disclosure.

### DESCRIPTION OF THE FIGURES

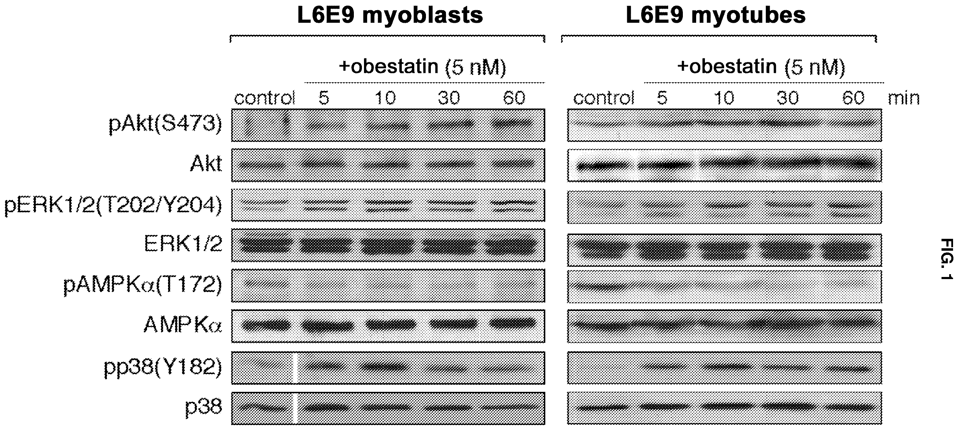

**Fig. 1****. Activation of regulatory enzymes of mitogenesis and myogenesis by obestatin in L6E9 myoblasts and myotubes.** Effect of obestatin on activity of regulatory enzymes of the mitogenic capacity (ERK 1/2), metabolic capacity (Akt and AMPK) and myogenic capacity (Akt and p38). Based on a dose-response study, the saturating dose for the maximum enzymatic activation is established for a concentration of 5 nM. The immunoblot analysis shows an activation profile for Akt, ERK 1/2 and p38 in myoblasts and myotubes. Inactivation of AMPK is described in both cell models. The activation profile discovers bimodal characteristics for the action of obestatin: 1.-mitogenic activation in myoblasts; and, 2.- myogenic activation. The immunoblots are representative of 6 independent assays.

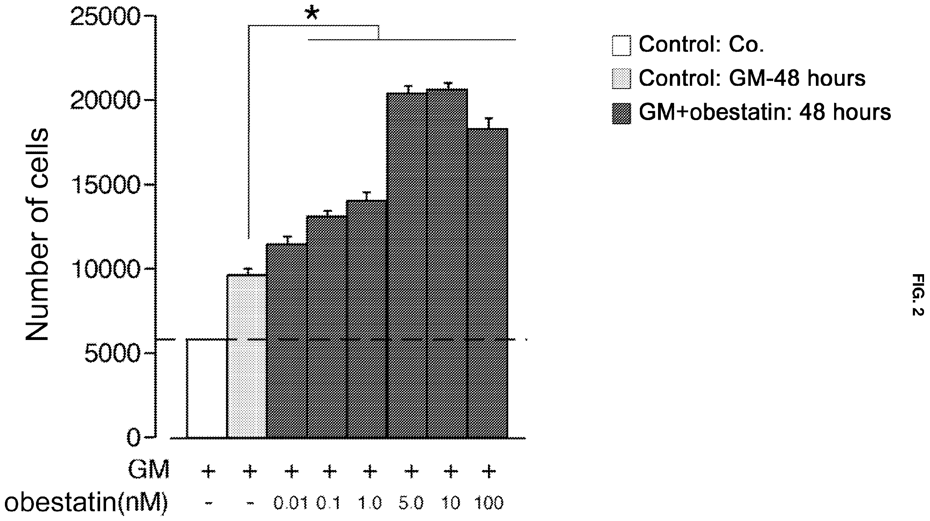

**Fig. 2****. Mitogenic effect of obestatin in L6E9 myoblasts.** Mitogenic effect of different doses of obestatin (0.01-100 nM) in L6E9 myoblasts after 48 hours of stimulation in the presence of growth medium [GM: DMEN+10% FBS]. Co: initial number of cells. Control: number of cells after 48 hours in GM. GM+obestatin: number of cells after 48 hours of stimulation with obestatin in GM. The data are expressed as mean±standard error of 4 independent experiments (*, p<0.05).

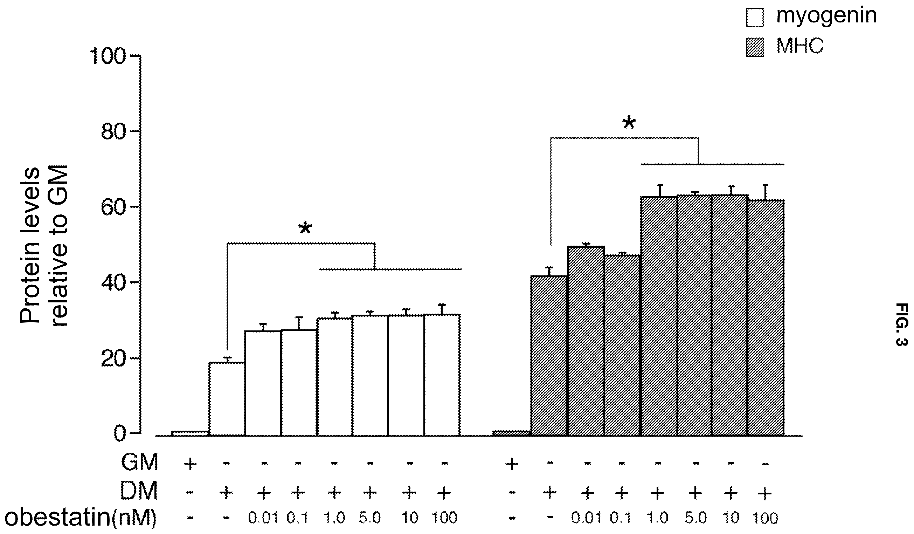

**Fig. 3****. Effect of obestatin on the expression of the myogenic markers myogenin and MHC in L6E9 myoblasts.** Effect of the stimulation with different doses of obestatin (0.01-100) on the expression of the myogenic markers myogenin and myosin (MHC) 6 days after the induction of the differentiation by the use of differentiation medium (DM: DMEN+2% FBS). The protein expression levels refer to the levels of myoblasts in growth medium (GM: DMEN+10% FBS), non-differentiated. The data are represented as % with respect to the cell expression in GM (mean±standard error of 6 independent experiments). The evaluation of the degree of expression was performed by immunoblot by the use of the ImageJ64 program (*, p<0.05).

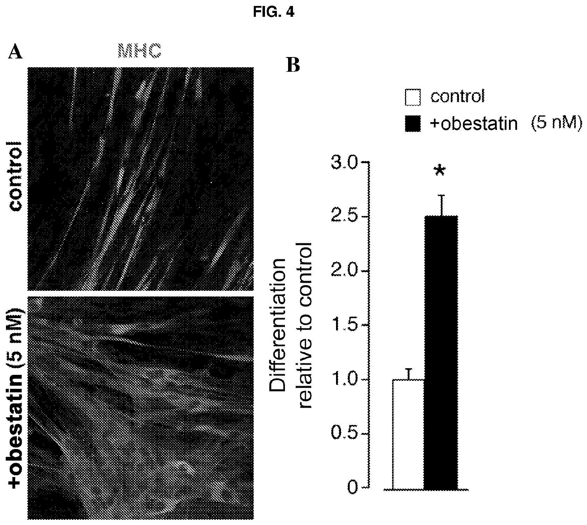

**Fig. 4****. Differentiation index associated to obestatin estimated in L6E9 myoblasts by immunofluorescence.** A: estimate of the myogenic capacity of obestatin (5 nM) by immunofluorescence (differentiation index). The cells were differentiated in DM (control) or in DM+obestatin (5 nM) during 6 days. Once fixed, the cells were stained with the anti-MHC antibody (40x magnification). B: quantification of the differentiation index of cells differentiated in DM (control) or in DM+obestatin (5 nM) during 6 days. The data are represented relative to the number of control cells positive for MHC (mean±standard error; *, p<0.05).

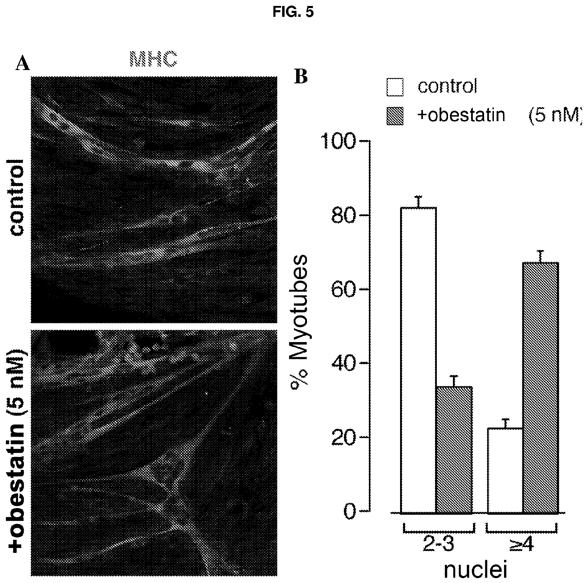

**Fig. 5****. Fusion index (number of nuclei per myotube) associated to obestatin estimated in L6E9 by immunofluorescence.** A: estimate of the myotube fusion capacity (fusion index) of obestatin (5 nM) by immunofluorescence. The cells were differentiated in DM (control) or in DM+obestatin (5 nM) during 6 days. Once fixed, the cells were stained with the anti-MHC antibody (40x magnification). B: quantification of the fusion index for the differentiated cells in DM (control) or in DM+obestatin (5 nM) during 6 days. The data are represented as % of the total number of cell positive for MHC with ≥ 2 nuclei (mean±standard error).

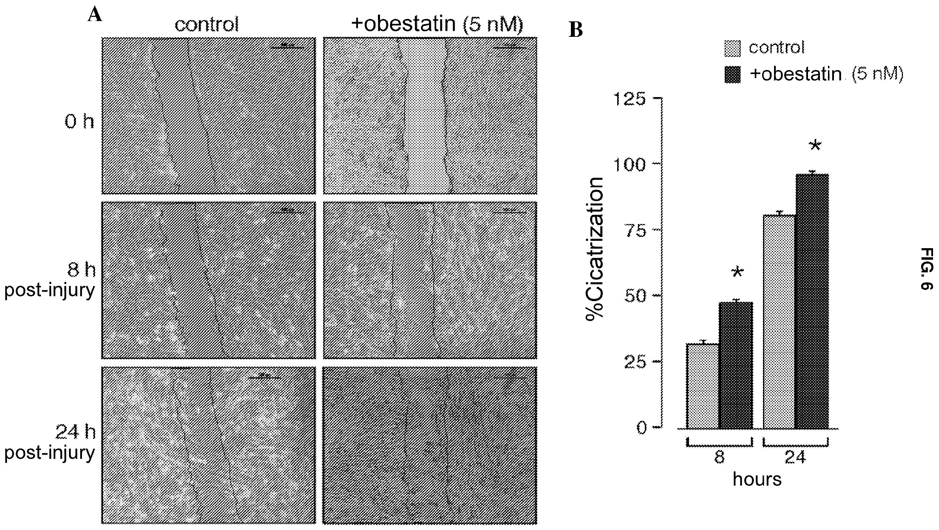

**Fig. 6****. Evaluation of the cicatrization capacity (migration/invasion) of obestatin in L6E9 myoblasts.** A: the L6E9 myoblasts grew to 100% of confluence in GM and later the damage was induced with a sterile pipette. The cells were maintained in GM or GM+obestatin (5 nM) during 8 and 24 hours after the induction of cell damage (40x magnification). B: quantification of the % of cicatrization (mean±standard error; *, p<0.05).

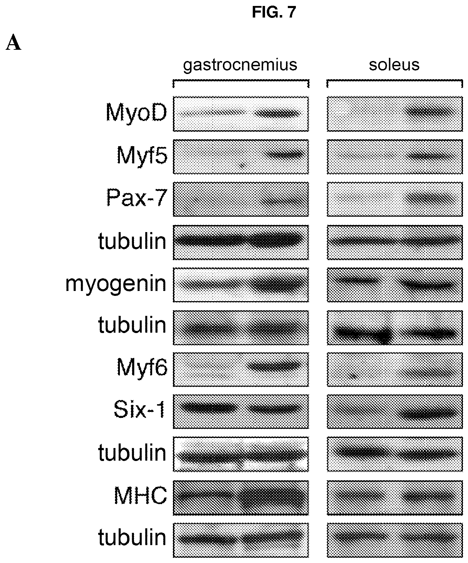

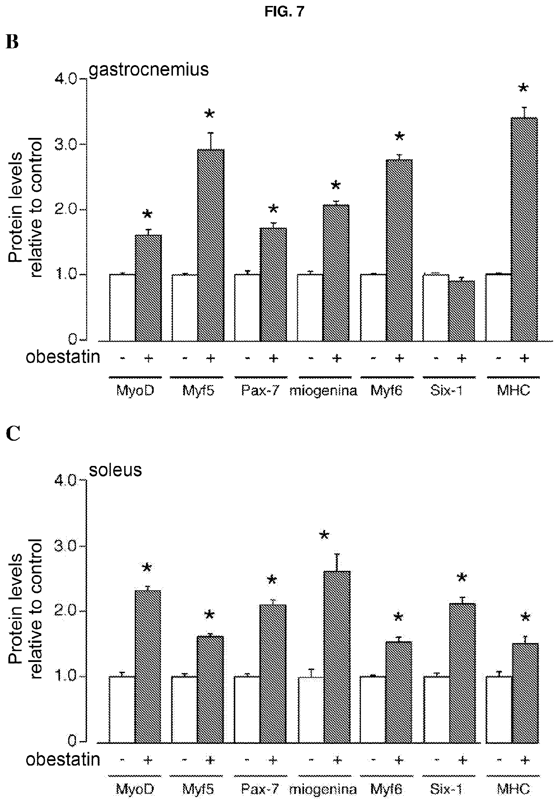

**Fig. 7****. Effect of the chronic administration of obestatin on the myogenic markers in the gastrocnemius and soleus of rat (animal model).** Effect of the administration during 72 hours of obestatin (300 nmol/kg/24h; 72 h) by the implantation of mini-pumps on early and later markers of myogenesis in skeletal muscle tissue (gastrocnemius and soleus) in male rats. A: Western-blot analysis. First column: control; second column: treatment with obestatin. B: Protein levels in gastrocnemius. White column: control; grey column: treatment with obestatin. The protein levels are expressed as % of the control obtained from the group of animals with implantation of mini-pumps with saline (n=10 per group; mean±standard error; *, p<0.05). C: Protein levels in soleus. White column: control; grey column: treatment with obestatin. The protein levels are expressed as % of the control obtained from the group of animals with implantation of mini-pumps with saline (n=10 per group; mean±standard error; *, p<0.05).

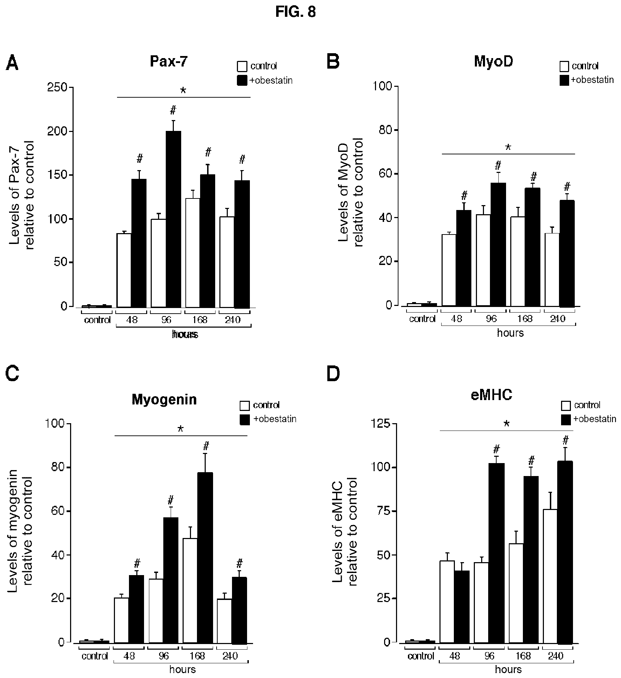

**Fig. 8****. Effect of the exogenous administration of obestatin in the damaged tibialis anterior of mouse (animal model) on the myogenic markers PAX-7 (A), MyoD (B), myogenin (C) and eMHC (D).** Effect of the local exogenous administration of obestatin (300 nmol/kg/24h) on early and later markers of myogenesis in damaged skeletal muscle tissue (tibialis anterior) in mice. The protein levels are expressed as % of the control obtained from the group of animals with administration of PBS (n=10 per group; mean ± standard error; *, #, p<0.005).

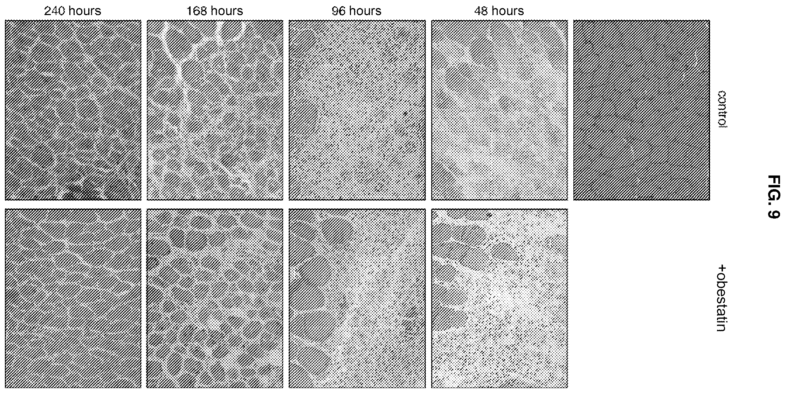

**Fig. 9****. Immunohistochemical analysis of the effect of the exogenous administration of obestatin on the capacity for muscle regeneration in the damaged tibialis anterior of mouse (animal model).** The images shown are representative of the sections of the damaged tibialis anterior muscle (hematoxylin/eosin staining) under the local exogenous administration of obestatin (300 nmol/kg/24h) or PBS (control) 48, 96, 168 and 240 h after inducing the damage. The images show the area of damage and regeneration at the indicated times.

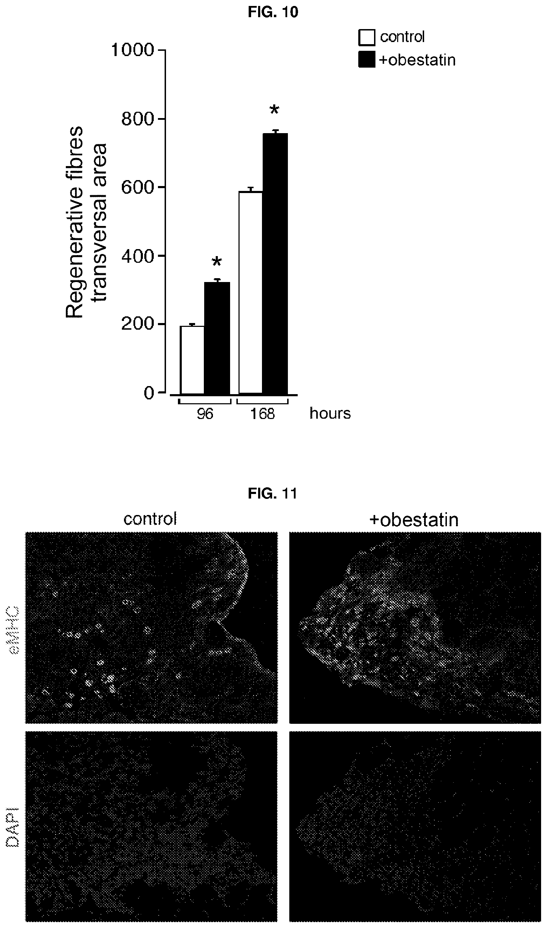

**Fig. 10****. Quantitative analysis of the areas of the regenerative fibres of the tibialis anterior 96 and 168 hours after inducing the damage and exogenous administration of obestatin or PBS (control).** The analysis corresponds to the area of regeneration myofibers in the tibialis anterior. For this purpose, an immunohistochemical analysis was performed by staining with hematoxylin/eosin and later quantification of the area of the myofiber by the use of the ImageJ64 program (n=10 per group; mean ± standard error; *, p<0.005).
**Fig. 11****.** Immufluorescence analysis of the effect of the exogenous administration of obestatin (96 h) on eMHC expression in the damaged tibialis anterior of mouse (animal model). Representative images of eMHC expression by immunofluorescence of sections of the tibialis anterior treated with obestatin (300 nmol/kg/24h) or PBS (control) 96 h after inducing the damage. The upper panels correspond to staining with anti-eMHC antibody and the lower panels to staining with DAPI.

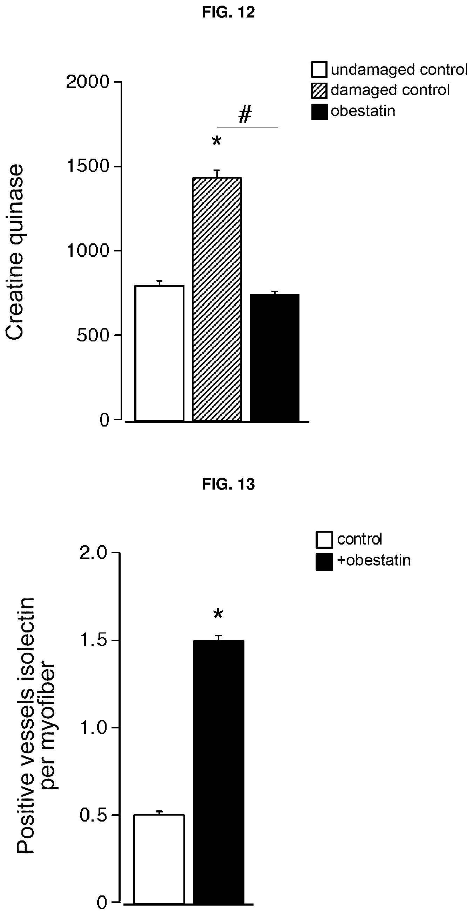

**Fig. 12****. Analysis of the creatine kinase levels in serum of mice wherein damage was induced in the tibialis anterior muscle and they were treated locally with obestatin or vehicle (PBS) during 96 h.** The creatine kinase levels are expressed as IU/L (International units/litre for the three study groups: control without damage, damage control (PBS) and treatment with obestatin (n=10 per group; mean ± standard error; *,#, p<0.005).
**Fig. 13****. Quantification of the blood vessels per myofiber in the tibialis anterior 10 days after the induction of muscle damage and exogenous treatment of obestatin (300 nmol/kg/24h) or vehicle (PBS).** Quantification of the number of blood vessels (immunofluorescence: positivity to isolectin) per myofiber (mean ± standard error; *, p<0.005) of sections of the tibialis anterior treated with obestatin (300 nmol/kg/24h) or PBS (control) 10 days after inducing the damage.

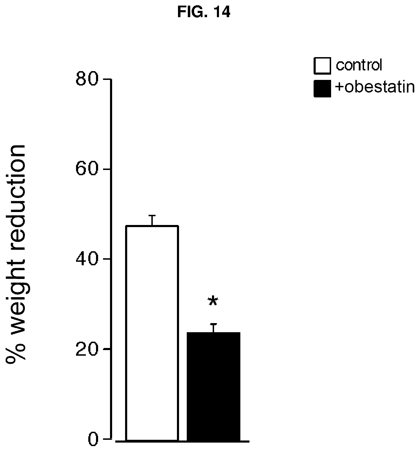

**Fig. 14****. Analysis of the weight reduction of the tibialis anterior muscle in mice where the sciatic nerve has been denervated (model of muscular atrophy) under the exogenous administration of obestatin or vehicle (PBS).** Swiss mice were used whereon the sciatic nerve was denervated in one of the limbs, using the other as control. The effect of the administration of obestatin (300 nmol/kg weight) or the vehicle (PBS; control) was assessed in the tibialis anterior muscle after14 days. The data are expressed as % of weight reduction with respect to the control without denervation (n=5 per group; mean ± standard error; *, p<0.005).

### EXAMPLES

Below, the invention is illustrated by tests performed by the inventors, revealing the efficacy of obestatin as myogenic agent and, therefore, as muscle regenerator. These specific examples provided serve to illustrate the nature of the present invention and are only included for illustrative purposes, so that they have not to be interpreted as limiting of the invention claimed herein. Therefore, the examples described below illustrate the invention without limiting the scope thereof.

### METHODS

**MATERIALS.** The rat/mouse obestatin was obtained from California Peptide Research (CA, USA). Anti-pAkt HM(S473), anti-Akt, anti-pERK1/2(T202/Y204), anti-ERK 1/2, anti-AMPKa(T172), anti-AMPKα, anti-pp38(Y182), anti-p38, anti-p21 and anti-tubulin antibodies were from Cell Signaling Technology (MA, USA). Anti-myosin heavy chain antibody (MHC) and anti-laminin antibody were obtained from Abcam (Cambridge, UK). Anti-myogenin, anti-MyoD, anti-Myf5, anti-Pax-7, anti-Myf6 and anti-Six-1 antibody were from Santa Cruz Biotechnology (CA, USA). The anti-isolectin GS-IB4 Alexa Fluor 594 antibody and goat anti-mouse antibody conjugated with FITC were obtained from Invitrogen (CA, USA). The anti-embryonic myosin heavy chain antibodies (eMHC) were obtained from Developmental Studies Hybridoma Bank (IA, USA). The secondary antibodies and the Enhanced Chemiluminescence Detection System were from Pierce (IL, USA). Alzet® osmotic mini-pumps (model 1003D) were acquired from Alzet Corporation (CA, USA). The other reagents were from Sigma-Aldrich (Mo, USA).

**CULTURE AND DIFFERENTIATION OF THE L6E9 MYOBLASTS.** The studies were performed based on the use of L6E9 rat myoblasts which were maintained in growth medium (GM) formed by DMEM (Dulbecco's Modified Eagle Medium) supplemented with 10% fetal bovine serum (FBS), 100 U/mL penicillin, and 100 U/mL streptomycin. For the differentiation, the cells were cultured at 80-100% of confluence in GM and it was replaced for differentiation medium (DM) formed by DMEM supplemented with 2% FBS, 100 U/mL penicillin, and 100 U/mL streptomycin during 6 days.

**IMMUNOBLOT ANALYSIS.** The tissue or cell samples were directly lysed in RIPA buffer [Tris-HCl (pH 7.2), 50 mM; NaCl, 150 mM; EDTA, 1 mM; NP-40, 1% (v/v); Sodium deoxycholate, 0.25% (w/v); protease inhibitors (Sigma); phosphatise inhibitors (Sigma)]. The lysates were clarified by centrifugation (14,000xg during 15 min at 4°C) and the protein concentration was quantified using QuantiPro™ BCA assay kit (Sigma). For immunoblot, equal amounts of protein were loaded which were fractionated in SDS-PAGE gels and transferred to nitrocellulose membranes. The immunoreactive bands were detected by chemiluminescence (Pierce ECL Western Blotting Substrate; Thermo Scientific, IL, USA).

**IMMUNOHISTOCHEMICAL ANALYSIS.** The muscle samples were mounted in a cold freezing medium [Tragacanth; Santa Cruz Biotechnology (CA, USA)] and frozen in a mixture of nitrogen-isopentane. The tissue sections were obtained by serial cuts with a cryostat (10 µm) and were mounted in adhesion slides for histochemistry (Marienfeld, Lauda-Konigshofen, Germany). The sections fixed were stained with hematoxylin/eosin. The quantification of the area of the regenerative myofibers was performed by using ImageJ64 analysis software.

**ANALYSIS BY IMMUNOFLUORESCENCE MICROSCOPY.** The L6E9 were cultured and differentiated on confocal microscope slides in DM supplemented or not with obestatin (5 nM) during 6 days. The cells were fixed using a buffered solution of PBS-paraformaldehyde during 15 min, washed, permeabilized and blocked with PBT [1% Triton X-100, 1% Tween-20, 5% inactivated serum, 0.2% BSA in PBS buffer] during 30 min, and later incubated with anti-MHC antibody diluted in PBT (1:400) during 12 h at 4°C. After three washes with PBS, the cells were incubated with the secondary antibody (goat anti-mouse antibody conjugate) in PBT (1:1000) during 45 min at 37°C. The DAPI was used to stain the nucleus. The digital images of the cell cultures were acquired with Leica TCS-SP2 confocal microscope. Five fields were taken of three independent experiments for each group. The degree of differentiation was evaluated based on the number of cells positive to MHC among the total number of nuclei. The number of nuclei within the myotubes (≥ 2 nuclei) was quantified for 20-50 myotubes. The myotubes were grouped in two categories, those with 2-3 nuclei and with 4 or more nuclei. The percentage of myotubes in each category was calculated relative to the total number of cells.

For the tissue immunofluorescence analysis, frozen sections were obtained which were fixed using a buffered solution of PBS-paraformaldehyde during 15 min, washed, permeabilized and blocked with PBT during 30 min, and later incubated with the corresponding primary antibody (anti-eMHC, anti-laminin antibody) diluted in PBT (1:50) during 12 h at 4°C. After three washes with PBS, the tissue sections were incubated with secondary antibody in PBT (1:1000) during 45 min at 37°C. The nuclei were displayed by staining with DAPI. The digital images of the cell culture were acquired with a Leica TCS-SP2 confocal microscope.

**INVASION/MIGRATION ASSAYS.** The L6E9 cells were cultured until reaching a confluence of 100% in GM. Having reached said confluence, the damage was produced by the use of a sterile pipette point which made it possible to perform two linear and perpendicular channels. After the damage, the cells were washed and cultured with GM or GM+obestatin (5 nM). The progress of the migration was immediately photographed after the damage and at 8 and 24 hours, close to the crossing point. The "invaded" area was calculated by tracing the "cicatrization" edge and using ImageJ64 software. The percentage of cicatrization was calculated based on the following equation:%cicatrization=[(area 0h-area xh)/ area 0 h]x100.

**ANIMAL MODELS.** Two animal models were used for the *in vivo* assays: Sprague-Dawley rats and Swiss mice. The Sprague-Dawley (250 g; n=20) rats were maintained in 12 h light-darkness cycled with free access to standard diet and water. The Alzet® mini-pumps (model 1003D) were subcutaneously implanted. The animals were assigned to one of the two experimental groups (n=10/group): 1) control group (mini-pumps with saline); and, 2) obestatin group (mini-pumps implanted containing 300 nmol/kg weight/24h). These mini-pumps allow a diffusion flow of 1 microL/h. After 72 h, the rats were sacrificed by cervical dislocation under anaesthetic treatment, and soleus and gastrocnemius tissues were extracted for immunoblot analysis.

The mice (30 g; n=50) were maintained in 12 h light-darkness cycled with free access to standard diet and water. To induce the muscle damage, the tibialis anterior muscle (TA) was extracted under anaesthesia and subjected to three cycles of damage due to cold contact applying along the length of the muscle a metal bar cooled in liquid nitrogen. The damaged muscles were treated locally with obestatin injection (300 nmol/kg weight) or the corresponding vehicle (PBS; control) during the times indicated in 24 h intervals. The animals were assigned to one of the five experimental groups (n=10/group): 1) control group (saline); 2) obestatin group 48 h (obestatin 300 nmol/kg weight/24h); 3) obestatin group 96 h (obestatin 300 nmol/kg weight/24h); 4) obestatin group 168 h (obestatin 300 nmol/kg weight/24h); and, obestatin group 240 h (obestatin 300 nmol/kg weight/24h). After the indicated times, the mice were sacrificed by cervical dislocation under anaesthetic treatment and the tissues were extracted for immunoblot and immunohistochemical analyses.

To study muscular atrophy, the sciatic nerve was denervated using Swiss mice. The denervation of the sciatic nerve was performed under anaesthesia, making an incision in the mid thigh on the lateral face of the rear right limb, separating the muscles and raising the sciatic nerve with surgical forceps. A portion of the nerve was isolated removing a fragment thereof. Finally the incision was closed. These animals were locally administered an injection of obestatin (300 nmol/kg weight) or the corresponding vehicle (PBS; control) in intervals of 24 h during 14 days.

### EXAMPLE 1. Dose-response study for the activation of mitogenic and myogenic enzymes in L6E9 myoblasts and myotubes.

To define the mitogenic and myogenic effect of obestatin, the activation capacity was studied of intracellular targets regulating proliferation (ERK1/2) and/or differentiation (p38 and Akt). In myoblasts, the activation of Akt [pAkt(S473)], p38 [pp38(Y138)] and ERK 1/2 [pERK1/2(T202/Y204)] was observed at concentrations of 0.1 nM obestatin, wherein the phosphorylation levels reached saturable values at a concentration of 5 nM. Figure 1 shows that the treatment with obestatin (5 nM) in L6E9 myoblasts produces a dose-dependent increase for pAkt(S473), pp38(Y138) and pERK1/2(T202/Y204), which reaches maximum levels at 10 min post-stimulation. Likewise, obestatin (5 nM) dephosphorylates the AMPKa in T172 [pAMPKa(T172)], inactivating this enzyme. Said activation dynamics was similar in L6E9 myotubes under stimulation with obestatin (5 nM). These data indicate possible mitogenic and myogenic capacity.

### EXAMPLE 2. Dose-dependent study for the mitogenic capacity of obestatin in L6E9 myoblasts.

Dose-dependent studies performed on myoblasts with different obestatin concentrations (0.01-100 nM) in GM (proliferative conditions) show a maximum increase in the number of cells at 48 hours post-stimulation at a concentration of 5.0 nM obestatin (~2.2-increased in relation to the control; Figure 2). This demonstrates the proliferative capacity of obestatin on myoblasts, the step prior to cell differentiation.

### EXAMPLE 3. Dose-response study for the expression of myogenic markers performed on L6E9 cells (rat myoblasts).

The L6E9 cells were cultured in DM complemented with different concentrations of obestatin in a range of 0.01-100 nM during six days. As shown in Figure 3, the protein levels of myogenin and MHC, detected by immunoblot, showed an increase in expression for the cells treated with obestatin, the maximum effect being observed at a concentration of 5 nM for myogenin expression (increase of -1.7 in relation to the control cells) and MHC (increase of -1.6 in relation to the control cells).

### EXAMPLE 4 and 5. Fluorescence microscopy studies for the estimate of the differentiation and fusion index for the treatment with obestatin in L6E9 cells.

The data show that the treatment with obestatin increases -150% the differentiation capacity in relation to the control cells (Figure 4). Furthermore, approximately 22% of the control myotubes had ≥4 nuclei, compared with -67% of the cells under treatment with obestatin (Figure 5). This effect represents an increase of -3.4 in the fusion index for treatment with obestatin.

### EXAMPLE 6. Evaluation of the cicatrization capacity (migration/invasion) of obestatin in L6E9 myoblasts.

The assays performed on L6E9 myoblasts to assess the migration index capacity (cicatrization) of obestatin (5 nM) show an increase of 53% 8 hours after inducing cell damage in relation to control cells (Figure 6). This increase is of 21% 24 hours after inducing cell damage. Working with short times allows eliminating the actual contribution of mitogenesis in the migration process.

### EXAMPLE 7. Study of expression of factors typical of mitogenesis in rats under the chronic administration of obestatin.

Based on the short mean life of obestatin in plasma, osmotic mini-pumps were used for the *in vivo* administration of this peptide. Two groups of male rats were worked with: a group of 10 male rats received the continuous subcutaneous infusion of obestatin during 72h (300 nmol/kg body weight/ 24h); and another group of 10 rats received the equivalent volume of PBS during 72h. The animals were sacrificed and the gastrocnemius and soleus muscles were removed and processed to analyse the expression of key factors of muscular differentiation by immunoblot. The levels of MyoD (Myogenic regulatory factor D), Myf5 (Myogenic regulatory factor 5), Pax-7 (Paired box protein 7), myogenin, Myf6 (Myogenic factor 6) and MHC were significantly increased in the gastrocnemius and soleus muscle of the rats under treatment with obestatin in relation to the group of control rats (Figure 7). Six-1 (Homeobox protein SIX1) showed a significant increased in the soleus samples, whilst no effect at all was observed in the gastrocnemius samples. The increase in both types of muscle of the family of regulatory factors of different stages in myogenesis show a clear muscle regenerative capacity for obestatin.

### EXAMPLE 8. Effect of the exogenous administration of obestatin in the damaged tibialis anterior of mouse (animal model) on the myogenic markers PAX-7 (A), MyoD (B), myogenin (C) and eMHC (D).

The estimate of the therapeutic potential of obestatin as muscle regenerator agent was assessed in Swiss mice wherein muscle damage was induced in the tibialis anterior and obestatin (300 nmol/kg weight) or the corresponding vehicle (PBS) was later locally administered. The muscles were extracted and analysed by immunoblot 48, 96, 168 and 240 h after the treatment (n=5 per study time). The levels of Pax-7, MyoD, myogenin and eMHC were significantly higher in the muscles treated with obestatin in relation to the control muscles (Figure 8). Furthermore, an acceleration in the expression times were observed for each one of the factors analysed.

### EXAMPLE 9. Immunohistochemical analysis of the effect of the exogenous administration of obestatin on the capacity for muscle regeneration in the damaged tibialis anterior of mouse (animal model).

The histological examination of the sections of the tibialis anterior muscle obtained during the muscle regeneration process showed a significant increase in the calibre of the regenerative myofibers in those animals treated with obestatin (300 nmol/kg weight) in relation to the corresponding controls. Furthermore, an acceleration could be verified in the regenerative process to judge by the restructuring of the myofibers in the area of damage at the different times in relation to the controls (Figure 9). These data are indicative of an increase in the regenerative capacity of the muscle.

### EXAMPLE 10. Quantitative analysis of the areas of the regenerative fibres of the tibialis anterior 96 and 168 hours after inducing the damage and exogenous administration of obestatin or PBS (control).

An analysis of the area of the regenerative fibres in the tibialis anterior showed a significant increase in those animals treated with obestatin (300 nmol/kg weight) in relation to the corresponding controls 96 and 168 h after the treatment (Figure 10). The increase in area of the regenerative myofibers together with the regeneration speed are indicative of an increase in muscle regeneration under treatment with obestatin.

### EXAMPLE 11. Immunofluorescence analysis of the effect of the exogenous administration of obestatin (96 h) on the expression of eMHC in the damaged tibialis anterior of mouse (animal model).

eMHC expression, which is absent in undamaged muscle, gradually increases during the regeneration process. The immunofluorescence analysis of eMHC expression in the tibialis anterior demonstrates that obestatin administration (300 nmol/kg weight; 96 h) significantly increases in the early stage of regeneration in relation to the controls (Figure 11). These data reinforce the results obtained for the differentiation markers analysed by immunoblot.

### EXAMPLE 12. Analysis of the creatine kinase levels in mice serum wherein damage was induced in the tibialis anterior muscle and they were treated locally with obestatin or vehicle (PBS) during 96 h.

Creatine kinase serum levels are associated with muscle damage. In animals wherein damage was induced in the tibialis anterior muscle and they were locally treated with obestatin (300 nmol/kg weight; 96 h) they showed a significant reduction in relation to the muscle damage controls under administration of the vehicle (PBS) (Figure 12). These data are indicative of a reduction in muscle damage due to the increase in the regenerative capacity under treatment with obestatin.

### EXAMPLE 13. Quantification of the blood vessels per myofiber in the tibialis anterior 10 days after the induction of muscle damage and exogenous treatment of obestatin (300 nmol/kg/24h) or vehicle (PBS).

The blood vessel count positive to isolectin in the tibialis anterior muscle shows a significant increase in capillary density after inducing muscle damage and treatment with obestatin (300 nmol/kg/24h) in relation to administration of the vehicle (PBS), indicating an increase in angiogenesis associated to the regenerative process (Figure 13). This is indicative of the fact that obestatin regulates the regeneration capacity and the supply of nutrients to the regeneration area.

### EXAMPLE 14. Analysis of the weight reduction of the tibialis anterior muscle in mice where the sciatic nerve has been denervated (model of muscular atrophy) under the exogenous administration of obestatin or vehicle (PBS).

An analysis of the weight of the tibialis anterior muscle in the animal model of muscular atrophy (denervation of the sciatic nerve) demonstrates that the local administration of obestatin (300 nmol/kg weight; 14 days) reduces the loss of muscle mass in relation to the control animals under treatment with the vehicle (PBS) (Figure 14). These data are indicative that obestatin is capable of counteracting the atrophic effects of the muscle through the increase in mass and, probably, muscle strength. This could be explained based on the regenerative and hypertrophic capacity demonstrated for said peptide.

## Claims

1. Use of an isolated peptide comprising an amino acid sequence comprising the SEQ ID NO: 3, or of the nucleotide sequence encoding it, as *in vitro* myogenic agent in skeletal muscle.

2. Use of the isolated peptide, or of the nucleotide sequence encoding it, according to claim 1, wherein said nucleotide sequence is included in an expression vector.

3. An isolated peptide comprising an amino acid sequence comprising the SEQ ID NO: 3, or the nucleotide sequence encoding it for use in the treatment and/or prevention of skeletal muscle injuries or skeletal muscle diseases selected from the list comprising: neurogenic myopathies, muscular necrosis, muscular atrophy, sprains, distensions, cramps, tendonitis, contractures, muscular dystrophy, myositis, muscle infections, myofascial pain, metabolic myopathies, muscle twitching, hypotonia, congenital myopathies, muscle paralysis, rhabdomyolysis or compartment syndrome.

4. The isolated peptide, or of the nucleotide sequence encoding it for use according to claim 3, wherein the skeletal muscle injuries or skeletal muscle diseases are selected from the list comprising: neurogenic myopathies, muscular necrosis, muscular atrophy or muscular dystrophy.

5. The isolated peptide, or the nucleotide sequence encoding it, for use according to any of claims 3 to 4, wherein said nucleotide sequence is included in an expression vector.

6. Pharmaceutical composition comprising:
a. an isolated peptide comprising an amino acid sequence comprising the SEQ ID NO: 3, or
b. the nucleotide sequence encoding the peptide of (a),
for use in the treatment and/or prevention of skeletal muscle injuries or skeletal muscle diseases selected from the list comprising: neurogenic myopathies, muscular necrosis, muscular atrophy, sprains, distensions, cramps, tendonitis, contractures, muscular dystrophy, myositis, muscle infections, myofascial pain, metabolic myopathies, muscle twitching, hypotonia, congenital myopathies, muscle paralysis, rhabdomyolysis or compartment syndrome.

7. Pharmaceutical composition for use according to claim 6, wherein the skeletal muscle injuries or skeletal muscle diseases are selected from the list comprising: neurogenic myopathies, muscular necrosis, muscular atrophy or muscular dystrophy.

## Patentansprüche

1. Verwendung eines isolierten Peptids, welches eine die SEQ ID NO: 3 umfassende Aminosäuresequenz umfasst, oder der Nukleotidsequenz, die es codiert, als In-vitro-Mittel für die Myogenese in der Skelettmuskulatur.

2. Verwendung des isolierten Peptids oder der Nukleotidsequenz, die es codiert, nach Anspruch 1, wobei die Nukleotidsequenz in einem Expressionsvektor enthalten ist.

3. Isoliertes Peptid, welches eine die SEQ ID NO: 3 umfassende Aminosäuresequenz umfasst, oder Nukleotidsequenz, die es codiert, zur Verwendung bei der Behandlung und/oder Vorbeugung von Verletzungen der Skelettmuskulatur oder Erkrankungen der Skelettmuskulatur, welche aus der Liste, umfassend: neurogene Myopathien, Muskelnekrose, Muskelatrophie, Verstauchungen, Distensionen, Krämpfe, Tendinitis, Kontrakturen, Muskeldystrophie, Myositis, Muskelinfektionen, myofasziale Schmerzen, metabolische Myopathien, Muskelzucken, Hypotonie, angeborene Myopathien, Muskellähmung, Rhabdomyolyse oder Kompartmentsyndrom ausgewählt sind.

4. Isoliertes Peptid oder Nukleotidsequenz, die es codiert, zur Verwendung nach Anspruch 3, wobei die Verletzungen der Skelettmuskulatur oder die Erkrankungen der Skelettmuskulatur aus der Liste, umfassend: neurogene Myopathien, Muskelnekrose, Muskelatrophie oder Muskeldystrophie ausgewählt sind.

5. Isoliertes Peptid oder Nukleotidsequenz, die es codiert, zur Verwendung nach einem der Ansprüche 3 bis 4, wobei die Nukleotidsequenz in einem Expressionsvektor enthalten ist.

6. Pharmazeutische Zusammensetzung, umfassend:
a. ein isoliertes Peptid, welches eine die SEQ ID NO: 3 umfassende Aminosäuresequenz umfasst, oder
b. die Nukleotidsequenz, welche das Peptid aus (a) codiert,
zur Verwendung bei der Behandlung und/oder Vorbeugung von Verletzungen der Skelettmuskulatur oder Erkrankungen der Skelettmuskulatur, welche aus der Liste, umfassend: neurogene Myopathien, Muskelnekrose, Muskelatrophie, Verstauchungen, Distensionen, Krämpfe, Tendinitis, Kontrakturen, Muskeldystrophie, Myositis, Muskelinfektionen, myofasziale Schmerzen, metabolische Myopathien, Muskelzucken, Hypotonie, angeborene Myopathien, Muskellähmung, Rhabdomyolyse oder Kompartmentsyndrom ausgewählt sind.

7. Pharmazeutische Zusammensetzung zur Verwendung nach Anspruch 6, wobei die Verletzungen der Skelettmuskulatur oder die Erkrankungen der Skelettmuskulatur aus der Liste, umfassend: neurogene Myopathien, Muskelnekrose, Muskelatrophie oder Muskeldystrophie ausgewählt sind.

## Revendications

1. Utilisation d'un peptide isolé comprenant une séquence d'aminoacides comprenant la SEQ ID NO : 3, ou de la séquence de nucléotides l'encodant, comme un agent myogénique *in vitro* dans le muscle squelettique.

2. Utilisation du peptide isolé, ou de la séquence de nucléotides l'encodant, selon la revendication 1, dans laquelle ladite séquence de nucléotides est incluse dans un vecteur d'expression.

3. Peptide isolé comprenant une séquence d'aminoacides comprenant la SEQ ID NO : 3, ou la séquence de nucléotides l'encodant en vue de son utilisation dans le traitement et/ou la prévention des blessures du muscle squelettique ou des maladies du muscle squelettique choisies dans la liste comprenant : myopathies neurogènes, nécrose musculaire, atrophie musculaire, entorses, distensions, crampes, tendinites, contractures, dystrophie musculaire, myosites, infections musculaires, douleurs myofasciales, myopathies métaboliques, contractions musculaires, hypotonie, myopathies congénitales, paralysie musculaire, rhabdomyolyse ou syndrome du compartiment.

4. Peptide isolé, ou de la séquence de nucléotides l'encodant en vue de son utilisation selon la revendication 3, dans lequel les blessures du muscle squelettique ou maladies du muscle squelettique sont choisies dans la liste comprenant : myopathies neurogènes, nécrose musculaire, atrophie musculaire ou dystrophie musculaire.

5. Peptide isolé, ou séquence de nucléotides l'encodant, en vue de son utilisation selon l'une quelconque des revendications 3 à 4, dans lequel ladite séquence de nucléotides est incluse dans un vecteur d'expression.

6. Composition pharmaceutique comprenant :
a. un peptide isolé comprenant une séquence d'aminoacides comprenant la SEQ ID NO : 3, ou
b. la séquence de nucléotides encodant le peptide de (a),
en vue de son utilisation dans le traitement et/ou la prévention des blessures du muscle squelettique ou des maladies du muscle squelettique choisies dans la liste comprenant : myopathies neurogènes, nécrose musculaire, atrophie musculaire, entorses, distensions, crampes, tendinites, contractures, dystrophie musculaire, myosites, infections musculaires, douleurs myofasciales, myopathies métaboliques, contractions musculaires, hypotonie, myopathies congénitales, paralysie musculaire, rhabdomyolyse ou syndrome du compartiment.

7. Composition pharmaceutique en vue de son utilisation selon la revendication 6, dans laquelle les blessures du muscle squelettique ou maladies du muscle squelettique sont choisies dans la liste comprenant : myopathies neurogènes, nécrose musculaire, atrophie musculaire ou dystrophie musculaire.
